Europäisches Patentamt

European Patent Office

Office européen des brevets

(11) Numéro de publication: **0 337 897**
**A1**

## (12) DEMANDE DE BREVET EUROPEEN

(21) Numéro de dépôt: **89420080.7**

(22) Date de dépôt: **28.02.89**

(51) Int. Cl.4: **C 04 B 35/64**
**A 61 K 6/06**

(30) Priorité: **14.03.88 FR 8803649**

(43) Date de publication de la demande:
**18.10.89 Bulletin 89/42**

(84) Etats contractants désignés:
**AT BE CH DE ES FR GB GR IT LI LU NL SE**

(71) Demandeur: **ETABLISSEMENTS DURAND - GIRARD**
**Société Anonyme**
**22-23 rue Juiverie**
**F-69005 Lyon (FR)**

(72) Inventeur: **Revel, François**
**5 quai des Etroits**
**F-69005 Lyon (FR)**

(74) Mandataire: **Maureau, Philippe et al**
**Cabinet GERMAIN & MAUREAU BP 3011**
**F-69392 Lyon Cédex 03 (FR)**

(54) **Procédé de cuisson de céramiques et composés métallocéramiques dentaires.**

(57) Ce procédé comporte, avant l'étape de cuisson proprement dite effectuée aux environs de 950°C (G), une étape préliminaire de maintien du produit à une température d'environ 130°C (E) pendant une durée variable selon le taux d'humidité du produit et se situant entre 1 et 10 minutes, ladite étape préliminaire étant suivie d'une étape de montée en température de 130°C à environ 650°C (F) pendant une durée variable selon la nature et/ou la granulométrie du produit et se situant entre 1°C et 250°C/minute, ladite étape de montée en température étant suivie d'une étape de maintien du produit à la température de calcination de 650°C (G) pendant 1 à 10 minutes.

Par ailleurs, l'étape de cuisson proprement dite est suivie d'une étape de refroidissement progressive comportant un palier de maintien sous atmosphère à une température d'environ 850°C (I) pendant trois minutes, suivie d'une étape de refroidissement linéaire jusqu'à une température basse d'environ 100°C.

FIG. 2

EP 0 337 897 A1

**Description**

## PROCEDE DE CUISSON DE CERAMIQUES ET COMPOSES METALLO-CERAMIQUES DENTAIRES

La présente invention concerne un procédé de cuisson de céramiques et composés métallo-céramiques dentaires.

On connaît toute l'importance que présentent, pour l'art dentaire, les matériaux céramiques et métallo-céramiques destinés tant à la réalisation de dents artificielles qu'au recouvrage total ou partiel des dents.

Il est absolument indispensable, pour des raisons esthétiques évidentes, que ces matériaux, une fois placés en bouche, présentent une teinte constante la plus proche possible des teintes des dents voisines et sans aucun phénomène indésirable opalescent.

Il convient, par ailleurs, que les céramiques présentent une parfaite compatibilité avec les alliages habituellement utilisés et notamment qu'il ne se produise pas de phénomènes de fissuration entre les différents éléments.

Lors des procédés classiques de cuisson des céramiques et composés métallo-céramiques dentaires, on fait subir au produit, avant l'opération de cuisson proprement dite, un traitement à l'aide de chaleur radiante et ceci dans le but d'exercer une déshydratation aussi complète que possible. Lors de ce traitement de déshydratation classique, le produit est porté, de façon continue et sensiblement linéaire, d'une température d'environ 100°C à la température de calcination qui se situe entre 550 et 650°C.

Cette façon de faire, très largement utilisée dans la pratique industrielle, peut toutefois occasionner certains inconvénients ; en effet, on soumet ensuite à l'opération de cuisson proprement dite (effectuée vers 950°C) un produit qui renferme encore des éléments organiques de fabrication tels que des colorants, des liquides de manipulation, etc...

Or, l'étape de cuisson proprement dite comporte une étape de montée en température régulière et sensiblement linéaire, qui conduit à un frittage de la surface de la céramique ; il en résulte un blocage des matières organiques à l'intérieur de la masse.

Ces matières organiques risquent de conduire ultérieurement à la formation de phénomènes de surface inesthétiques : microporosités, opalescence... tout à fait inacceptables pour des utilisations dans le domaine dentaire.

Par ailleurs, les procédés classiques connus jusqu'alors comportent, une fois le cycle de cuisson terminé, une étape de refroidissement continue, régulière, elle aussi pratiquement linéaire.

On a pu déterminer qu'une étape de refroidissement ainsi conduite comportait plusieurs inconvénients notamment quant aux dangers de fissuration des liaisons métal-céramique.

Le procédé selon l'invention s'est donné pour objet de remédier aux inconvénients de la technique antérieure exposés ci-avant en évitant, d'une part, tout risque d'inclusion de matières organiques à l'intérieur du produit céramique ou métallo-céramique et, d'autre part, en minimisant les dangers de fissuration du complexe céramique-métal.

L'Inventeur a pu déterminer qu'il était possible d'atteindre ce but en ménageant, dans la courbe de montée jusqu'à la température de calcination et de descente de la température de cuisson à la température basse, un ou plusieurs paliers au cours desquels est maintenue une température constante, ceci étant rendu possible par une programmation soigneuse du processus de cuisson.

Le procédé de cuisson de composés céramiques et métallo-céramiques dentaires selon l'invention est caractérisé en ce qu'il comporte, avant l'étape de cuisson proprement dite effectuée aux environs de 950°C, une étape préliminaire de maintien du produit à une température d'environ 130°C pendant une durée variable selon le taux d'humidité du produit et se situant entre 1 et 10 minutes, ladite étape préliminaire étant suivie d'une étape de montée en température de 130°C à environ 650°C pendant une durée variable selon la nature et/ou la granulométrie du produit et se situant entre 1°C et 250°C/minute, ladite étape de montée en température étant suivie d'une étape de maintien du produit à la température de calcination de 650°C pendant 1 à 10 minutes.

Cette modification du procédé classique assure une déshydratation régulière du produit, en même temps qu'une élimination progressive des matières organiques susceptibles de créer des phénomènes inesthétiques dans le produit fini.

Avantageusement, et selon une autre caractéristique de l'invention, l'étape de cuisson proprement dite est suivie d'une étape de refroidissement progressive comportant un palier de maintien sous atmosphère à une température d'environ 850°C pendant environ 3 minutes, suivie d'une étape de refroidissement linéaire jusqu'à une température basse d'environ 100°C.

Cette étape de refroidissement progressive permet de réaliser une cristallisation secondaire du produit céramique en augmentant son réseau dendritique ; grâce à cette cristallisation, on maintient le coefficient d'expansion thermique du produit céramique à un niveau voisin du coefficient d'expansion thermique de l'alliage dentaire auquel il est associé, ce qui réduit les dangers de fissuration.

L'ensemble des opérations du procédé de cuisson selon l'invention est avantageusement effectué dans un four muni d'un programme de régulation.

La présente invention sera mieux comprise et ses avantages ressortiront bien de la description qui suit en référence au dessin schématique annexé dans lequel :

Figure 1 est une représentation graphique d'un cycle de cuisson classique d'une céramique dentaire ;

Figure 2 est une représentation graphique illustrant les étapes du processus de cuisson selon l'invention, telles qu'elles sont déterminées par un programme spécialement mis au point.

Sur les figures, les températures sont portées en ordonnées.

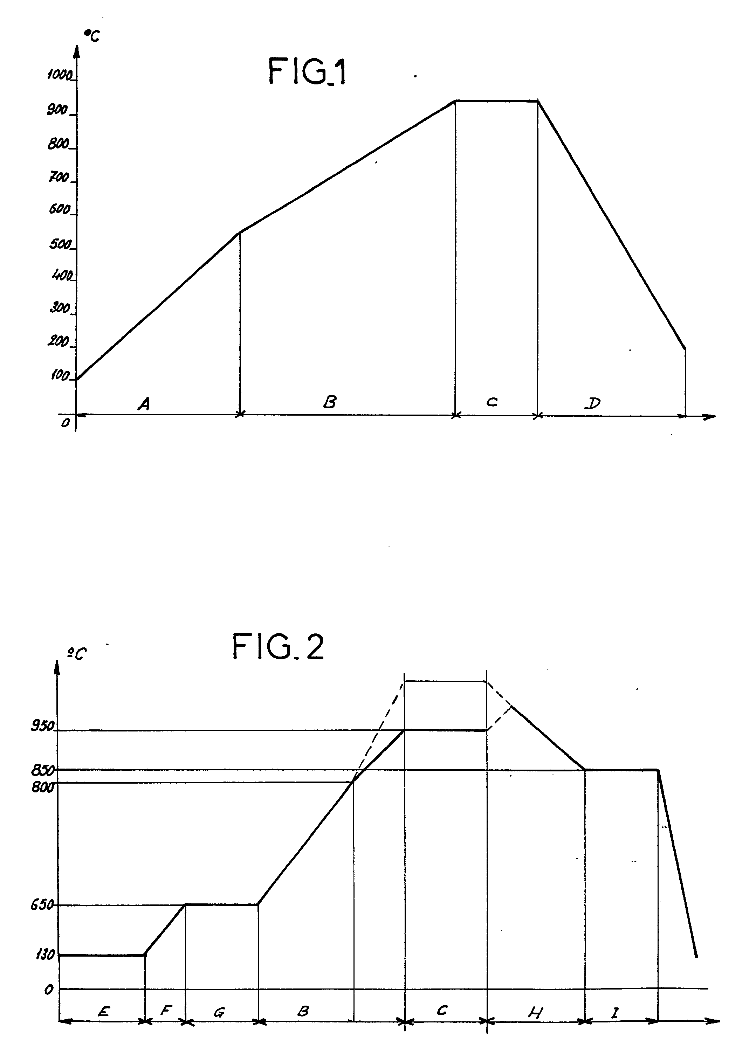

On voit, sur le graphique représenté à la figure 1 que, dans le procédé classique de cuisson, la céramique, introduite dans le four porté à une température d'environ 100°C, est régulièrement portée à la température de calcination, entre 550 et 650°C.

Cette montée en température s'effectue de façon pratiquement linéaire, et dure environ quatre minutes.

C'est au cours de cette étape (partie A du graphique) que doit s'effectuer la déshydratation de la céramique, avec les défauts mentionnés ci-avant et notamment le blocage des matières organiques (colorants, liquides de manipulation...) à l'intérieur de la céramique.

Cette étape A de déshydratation est suivie de l'étape B de cuisson proprement dite, qui s'effectue le plus souvent sous vide et pendant laquelle la céramique est portée aux environs de 950°C à une vitesse d'environ 55°C/minute.

Toujours de façon classique, l'étape B de cuisson est suivie d'une étape C de stabilisation à la température de cuisson, aux environs de 950°C, sous atmosphère, pendant environ 1 minute.

Enfin, la dernière étape D du procédé classique est une étape de refroidissement dans laquelle la température du four est abaissée régulièrement, de façon sensiblement linéaire, jusqu'à une température d'environ 200°C.

Cette étape de refroidissement linéaire peut conduire à des problèmes de fracture, notamment dus aux différences des coefficients d'expansion thermique entre la céramique et l'alliage dentaire.

Le procédé selon l'invention va maintenant être décrit en référence à la figure 2, qui illustre graphiquement, de façon très schématique, et notamment sans que soit respectée l'échelle des températures, un programme spécialement mis au point pour assurer la régulation des fours de cuisson utilisés pour la mise en oeuvre dudit proécé.

Le four est, tout d'abord, programme à température basse (environ 130°C).

On programme la durée de montée du plateau entre 1 à 10 minutes, puis le maintien dudit plateau à cette température d'environ 130°C pendant une durée pouvant aller de 1 à 10 minutes, suivant la qualité de la ceramique (étape E du graphique).

Cette étape, tout à fait décisive du procédé selon l'invention, a pour but de parfaire la déshydratation de la céramique.

Le programme comprend ensuite la programmation de la vitesse de montée depuis la température basse de 130°C jusqu'à la température de calcination, aux environs de 650°C, cette vitesse pouvant se situer toujours suivant la qualité de la céramique entre 1°C/minute et 250°C/minute ; la montée en température dans cette étape F du graphique se fait sous atmosphère.

Il est prévu, selon une autre caractéristique importante du procédé, une possibilité de maintien à cette température d'environ 650°C pendant une à six minutes (étape G) ; ce palier à la température de calcination peut se faire sous atmosphère ou sous vide.

Les étapes suivantes correspondent aux étapes B et C du processus de cuisson classique.

Le programme comprend alors la programmation de la vitesse de montée en température depuis la température de calcination (environ 650°C jusqu'à la température finale d'environ 950°C ($\pm$ 10°C selon que l'étape de cuisson proprement dite se fera sous vide ou sous atmosphère) (étape B).

Si l'on travaille sous vide, le niveau de vide est programmé d'environ $1,33 \times 10^{-2}$ Pa à environ $997 \times 10^{-2}$ Pa de même que le palier de maintien sous vide à température finale (environ 950°C) (étape C).

Le palier de maintien à température finale (étape C) peut également se faire sous atmosphère (portée en pointillés et traits minces), la programmation étant effectuée en conséquence.

L'étape de refroidissement de la céramique (étape H) est maintenant amorcée, sous atmosphere et le programme prévoit l'introduction (étape 1) d'un palier réglable de maintien sous atmosphère à une température donnée (850°C pour le cas choisi).

C'est l'observation de ce palier qui permettra, selon une caractéristique importante de l'invention, de réaliser une cristallisation secondaire de la céramique pour augmenter son réseau dentritique, et ainsi de permettre que le coefficient d'expansion thermique de la céramique ne descende pas en-dessous de celui de l'alliage métallique, ce qui est très souvent le cas quand l'étape de refroidissement après cuisson est effectuée de façon linéaire (étape D de la figure 1). On évite ainsi le danger de fractures entre les deux éléments.

La descente du plateau est ensuite programmée de telle sorte que le refroidissement se poursuive de façon linéaire jusqu'à la température basse choisie d'environ 200°C (durée de 1 à 25 minutes).

## Revendications

1- Procédé de cuisson de composés céramiques et métallo-céramiques dentaires, caractérisé en ce qu'il comporte, avant l'étape de cuisson proprement dite effectuée aux environs de 950°C, une étape préliminaire de maintien du produit à une température d'environ 130°C pendant une durée variable selon le taux d'humidité du produit et se situant entre 1 et 10 minutes, ladite étape préliminaire étant suivie d'une étape de montée en température de 130°C à environ 650°C pendant une durée variable selon la nature et/ou la granulométrie du produit et se situant entre 1°C et 250°C/minute, ladite étape de montée en température étant suivie d'une étape de maintien du produit à la température de calcination de 650°C pendant 1 à 10 minutes.

2- Procédé selon la revendication 1, caractérisé en ce que l'étape de cuisson proprement dite est suivie d'une étape de refroidissement progressive comportant un palier de maintien sous atmosphère a une température d'environ 850°C pendant environ 3 minutes, suivie d'une étape de refroidissement linéaire jusqu'à une température basse d'environ 100°C.

FIG.1

FIG.2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | DE-A-3 231 546  (DENTSPLY INTERNATIONAL INC.) <br> * Revendications 1-3; page 8, paragraphe 4 - page 10, paragraphe 1 * <br> --- | 1,2 | C 04 B  35/64 <br> A 61 K   6/06 |
| A | FR-A-2 307 514  (J. SANTIAGO) <br> * Revendication 1; page 1, lignes 19-32 * <br> ----- | 1,2 | |

DOMAINES TECHNIQUES
RECHERCHES (Int. Cl.4)

C 04 B
A 61 K

Le présent rapport a été établi pour toutes les revendications

| Lieu de la recherche | Date d'achèvement de la recherche | Examinateur |
|---|---|---|
| LA HAYE | 07-06-1989 | LUETHE H. |